# EUROPÄISCHE PATENTSCHRIFT

(11) **EP 1 514 575 B1**
(45) Veröffentlichungstag und Bekanntmachung des Hinweises auf die Patenterteilung: **30.05.2007**
(21) Anmeldenummer: 04018873.2
(22) Anmeldetag: 09.08.2004
(51) Int. Cl.: A61M 39/16, A61M 39/10

(54) **Vorrichtung zum Reinigen von Luer-Lock-Anschlüssen**
Device for cleaning Luer-Lock connectors
Dispositif pour nettoyer des connecteurs de type Luer-Lock

(30) Priorität: 11.09.2003 DE 10342237
(43) Veröffentlichungstag der Anmeldung: 16.03.2005
(73) Patentinhaber: BHT Hygienetechnik GmbH, 86368 Gersthofen (DE)
(72) Erfinder: Annecke, Karl Heinz, Dr, 64625 Bensheim (DE); Bayer, Wolfgang, 86551 Aichach (DE)
(74) Vertreter: von Bülow, Tam

(56) Entgegenhaltungen:
- DE-A- 10 303 381
- DE-U- 29 821 739
- US-A- 5 058 619
- US-A- 5 190 534

## Beschreibung

Die vorliegende Erfindung bezieht sich auf einen medizinischen Gegenstand in Form eines Luer-Lock-Gegenstückes, das ein Außengewinde aufweist, und eine Vorrichtung zum Reinigen desselben.

Medizinische Geräte, wie z.B. Spritzen, Kanülen, Infusionsleitungen, haben sog. Luer-Lock-Verbinder, wie sie beispielsweise in der DE 103 03 381 A1 gezeigt sind. Zum Reinigen und Desinfizieren dieser medizinischen Geräte werden sie in eine Reinigungsmaschine gelegt und an einen dort befindlichen Luer-Lock-Anschluß angeschlossen, der seinerseits mit einer Zufuhr für Reinigungsflüssigkeit, beispielsweise der Umwälzpumpe der Maschine verbunden ist. Hierzu ist es bisher üblich, als maschinenseitige Komponente des Luer-Lock-Verbinders die normale Komponente einer Luer-Lock-Verbindung zu verwenden.

Luer-Lock-Verbindungen haben eine sog. männliche und weibliche Komponente. Die männliche Komponente hat einen Luer-Kegel, der mit einem Konussitz in die sog. weibliche Komponente eingreift. Der Luer-Kegel hat dabei einen inneren Kanal und einen den Luer-Kegel umgebenden zylindrischen Mantel, der über einen scheibenförmigen Boden mit dem Luer-Kegel verbunden ist. Zwischen dem Luer-Kegel und dem Mantel ist damit ein Hohlraum gebildet, in den das Luer-Lock-Gegenstück teilweise eingreift. Dieser Mantel hat an seiner Innenseite, also in dem Hohlraum ein Innengewinde und das entsprechende Gegenstück ein dazu passendes Außengewinde.

Üblicherweise ist in der Reinigungsmaschine der Luer-Lock-Verbinder im wesentlichen senkrecht angeordnet, so daß die zu reinigenden Gegenstände vertikal von oben anschließbar sind.

Nachteilig hieran ist, daß in dem genannten Hohlraum auch nach Beendigung des Reinigungsvorganges Flüssigkeit stehen bleibt, die noch Verunreinigungen aufweist, so daß die Gefahr einer Kontamination besteht. Auch ist es problematisch, das Außengewinde des zu reinigenden Luer-Lock-Gegenstückes zu reinigen, da dort nur von "außen" Reinigungsflüssigkeit hingelangt, da der Luer-Kegel, über dessen inneren Kanal die Reinigungsflüssigkeit zugeführt wird, flüssigkeitsdicht an den inneren Kanal des Luer-Lock-Gegenstückes angeschlossen ist.

Die DE 298 21 739 U1 zeigt eine Vorrichtung zur Reinigung medizinischer Geräte mit einem zylindrischen Körper, in den ein zu reinigender medizinischer Gegenstand einsetzbar ist. Der zylindrische Körper hat mehrere in ihn mündende Kanäle, über die Reinigungsflüssigkeit zu- und abführbar ist. Zur Halterung des medizinischen Gerätes kann ein Adapter oder ein Gewinde vorgesehen sein. Da beim Einführen des medizinischen Gerätes in den Innenraum des zylindrischen Körpers, dessen obere Kante kontaminiert werden kann, ist ein Überlauf vorgesehen, so daß diese Kante von Reinigungsflüssigkeit überspült wird. Der Überlauf kann eine Auffangeinrichtung für überströmende Flüssigkeit aufweisen.

Die US 6,286,179 B1 zeigt eine Vorrichtung zum Reinigen von Endoskopen, bei der der Endoskopkopf dichtend in einen zylindrischen Hohlraum eingeführt wird, der zwei Zuläufe für Reinigungsflüssigkeit hat.

Die DE 31 43 329 A1 zeigt eine Schutzvorrichtung für Verbindungsstöpsel mit einem abgedichteten Hohlraum.

Aufgabe der Erfindung ist es daher, die Vorrichtung zum Reinigen von Luer-Lock-Anschlüssen dahingehend zu verbessern, daß ein besseres Reinigungsergebnis erzielt wird. Dies bedeutet insbesondere, daß nach Beendigung des Reinigungsvorganges sämtliche Flüssigkeit abgeführt werden kann und weiter, daß auch die Gewinde des Luer-Lock-Anschlusses einwandfrei gereinigt werden.

Diese Aufgabe wird durch die im Patentanspruch 1 angegebenen Merkmale gelöst. Vorteilhafte Ausgestaltungen und Weiterbildungen der Erfindung sind den Unteransprüchen zu entnehmen.

Das Grundprinzip der Erfindung besteht darin, daß der Kanal des maschinenseitigen Luer-Lock-Anschlusses bei angeschlossenem Luer-Lock-Gegenstück mit dem Innenraum des zylindrischen Mantels in Strömungsverbindung steht. Hierdurch wird während des Reinigungsvorganges über den Kanal auch Reinigungsflüssigkeit zu dem Gewinde der Luer-Lock-Verbindung gefördert und andererseits kann nach Beendigung des Reinigungsvorganges, wenn die Zufuhr von Reinigungsflüssigkeit beendet ist, also beispielsweise die Umwälzpumpe abgeschaltet ist, sämtliche Flüssigkeit von dem Innenraum abfließen.

Generell ist bei der Erfindung der Anschluß der beiden Luer-Lock-Komponenten in gewissem Maße flüssigkeitsdurchlässig. Es erfolgt also gerade keine absolut dichte Verbindung der beiden Komponenten, wie bei einem herkömmlichen Luer-Lock-Anschluß, sondern es wird gezielt eine Strömungsverbindung geschaffen, die eine weitgehende Spülung der gesamten Kupplungsstelle und einen Rücklauf von Restwasser gewährleistet. Andererseits ist selbstverständlich die Verbindung zwischen den beiden Komponenten so hergestellt, daß auch ein Flüssigkeitsdruck in das angekuppelte Luer-Lock-Gegenstück übertragen wird.

Nach einer Variante der Erfindung hat der Luer-Kegel des maschinenseitigen Anschlusses eine Öffnung zum inneren Kanal, so daß dieser in Strömungsverbindung mit dem Innenraum des zylindrischen Mantels steht. Während des Reinigungsvorganges gelangt also unter Druck stehende Reinigungsflüssigkeit durch diese Öffnung auch in den Innenraum des zylindrischen Mantels und damit an die Außenfläche des Luer-Kegels und von dort zu dem Gewinde des Luer-Lock-Gegenstückes. Beim Abschalten der Umwälzpumpe kann über diese Öffnung Restflüssigkeit über den Kanal abfließen.

Alternativ oder kumulativ hierzu kann auch der zylindrische Mantel eine radiale Öffnung haben, die möglichst nahe dem Boden des zylindrischen Mantels angeordnet ist, so daß hierüber ebenfalls Restflüssigkeit abfließen kann.

Es können auch mehrere solcher Öffnungen vorhanden sein, die in Längsrichtung des zylindrischen Mantels verteilt angeordnet sind.

Nach einem zweiten Ausführungsbeispiel der Erfindung mündet der innere Kanal des maschinenseitigen Luer-Lock-Anschlusses unmittelbar in den Hohlraum des zylindrischen Mantels. Mit anderen Worten wird am maschinenseitigen Luer-Lock-Anschluß der Luer-Kegel fortgelassen. Somit gelangt Reinigungsflüssigkeit vom inneren Kanal unmittelbar in den Innenraum des Mantels und von dort sowohl zur Innen- als auch zur Außenseite des Luer-Lock-Gegenstückes. Die Öffnungen im Mantel können bei diesem Ausführungsbeispiel fortgelassen sein. Sie können aber auch beibehalten werden.

Im folgenden wird die Erfindung anhand von Ausführungsbeispielen im Zusammenhang mit der Zeichnung ausführlicher erläutert. Es zeigt:
- Fig. 1: einen Längsschnitt eines maschinenseitigen Luer-Lock-Anschlusses mit angeschlossenem Luer-Lock-Gegenstück;
- Fig. 2: eine perspektivische Ansicht eines maschinenseitigen Luer-Lock-Anschlusses; und
- Fig. 3: ein zweites Ausführungsbeispiel eines maschinenseitigen Luer-Lock-Anschlusses mit angeschlossenem Luer-Lock-Gegenstück.

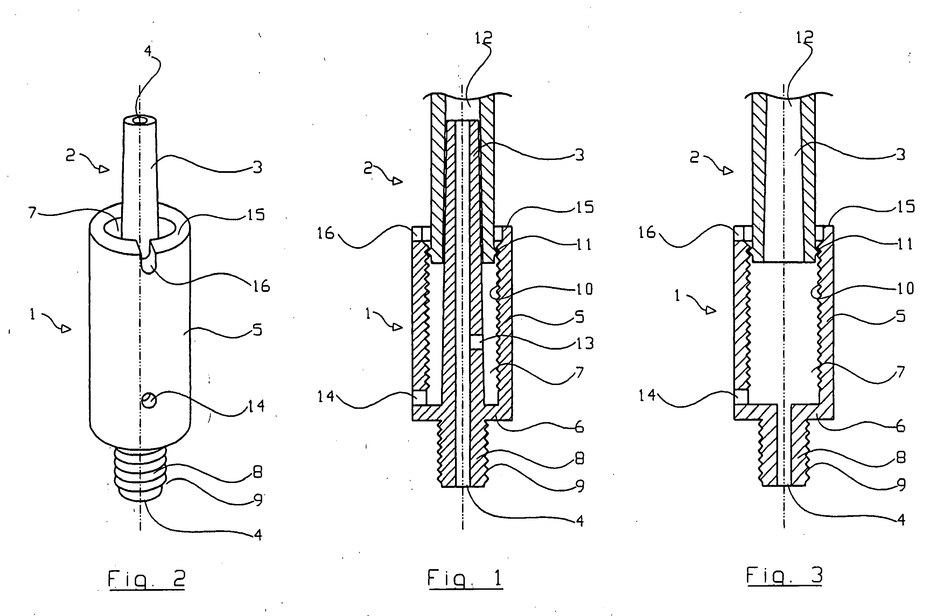

Fig. 1 zeigt einen maschinenseitigen Luer-Lock-Anschluß 1, an den ein zu reinigendes Luer-Lock-Gegenstück 2 angeschlossen ist, welches mit einem zu reinigenden Gegenstand, wie z.B. einer Spritze, einem Infusionsbesteck oder ähnlichem verbunden ist. Der maschinenseitige Luer-Lock-Anschluß 1 hat einen Luer-Kegel 3, durch den hindurch sich ein innerer Kanal 4 erstreckt, der an eine nicht dargestellte Zufuhr für Reinigungsflüssigkeit, wie z.B. eine Umwälzpumpe einer Reinigungsmaschine, angeschlossen ist. Rings um einen Abschnitt des Luer-Kegels 3 ist ein zylindrischer Körper 5 mit einem scheibenförmigen Boden 6 angeordnet, so daß sich zwischen dem Luer-Kegel 3 und der Innenwandung des zylindrischen Körpers 5 ein ringförmiger Hohlraum 7 ergibt, der zur Spitze des Luer-Kegels 3 hin offen ist. Auf der der Spitze des Luer-Kegels 3 abgewandten Seite des Bodens 6 ist ein Anschlußstutzen 8 mit einem Außengewinde 9 angebracht, womit der Luer-Lock-Anschluß 1 im Inneren der Reinigungsmaschine, beispielsweise an einem Einsatzkorb, anschließbar ist. Der Luer-Kegel 3, der zylindrische Körper 5, der Boden 6 und der Anschlußstutzen 9 können einstückig ausgebildet sein. Die Innenwandung des zylindrischen Körpers 5 weist ein Innengewinde 10 auf, in das ein Außengewinde 11 des Luer-Lock-Gegenstückes 2 einschraubbar ist. Das Luer-Lock-Gegenstück 2 hat einen inneren Kanal 12, dessen Wandung an den Luer-Kegel 3 angepaßt ist, so daß sich dort ein dichter Konussitz ergibt.

Der Luer-Kegel 3 weist eine radiale Öffnung 13 auf, die den inneren Kanal 4 mit dem ringförmigen Hohlraum 7 verbindet. Durch diese Öffnung gelangt Reinigungsflüssigkeit vom Kanal 4 in den Hohlraum und erreicht damit auch die Gewinde 10 und 11, die damit ebenfalls gereinigt werden. Da die Gewinde 10 und 11 nicht flüssigkeitsdicht ineinander greifen, ist also die Verbindung zwischen dem maschinenseitigen luer-Lock-Anschluß 1 und dem Luer-Lock-Gegenstück 2 ebenfalls nicht flüssigkeitsdicht. Beim Abschalten der Umwälzpumpe kann die Reinigungsflüssigkeit über die Kanäle 12 und 14 zurückfließen, wobei Flüssigkeit in dem Hohlraum 7 ebenfalls über die Öffnung 13 abfließen kann.

Weiter hat der zylindrische Körper 5 nahe dem Boden 6 eine radiale Öffnung 14, über die Flüssigkeit aus dem Hohlraum 7 abfließen kann.

Am oberen Rand 15 des zylindrischen Körpers 5 ist eine weitere radiale Öffnung 16 vorgesehen, die hier als nach oben offener Einschnitt ausgebildet ist. Über diese Öffnung kann ebenfalls Flüssigkeit, die sonst von oben her durch die Gewinde 10, 11 in den Hohlraum 7 eindringen würde, abfließen.

Das Ausführungsbeispiel der Fig. 3 unterscheidet sich von dem der Figuren 1 und 2 im wesentlichen dadurch, daß der maschinenseitige Luer-Lock-Anschluß 1 keinen Luer-Kegel mehr aufweist sondern nur noch den zylindrischen Körper 5 mit Innengewinde 10, in das das Außengewinde 11 des Luer-Lock-Gegenstückes 2 einschraubbar ist. Die beiden Luer-Lock-Komponenten 1 und 2 sind somit nur noch über die Gewinde 10 und 11 miteinander verbunden, so daß auch hier der innere Kanal 4 unmittelbar mit dem Innenraum 7 des zylindrischen Körpers 5 in Verbindung steht.

Die radialen Öffnungen 14 und 16 am zylindrischen Körper 5 können zwar noch vorgesehen sein. Sie sind aber nicht notwendig, da Restwasser aus dem Hohlraum 7 vollständig durch den Kanal 4 abfließen kann.

## Patentansprüche

1. Medizinischer Gegenstand in Form eines Luer-Lock-Gegenstückes (2), das ein Außengewinde (11) aufweist, und Vorrichtung zum Reinigen desselben, wobei die Vorrichtung einen zylindrischen Körper (5) aufweist,
**dadurch gekennzeichnet,**
**daß** die Vorrichtung (1) einen in den zylindrischen Körper (5) mündenden Kanal (4) aufweist, über den Reinigungsflüssigkeit zuführbar ist, wobei der Kanal (4) mit dem Innenraum des zylindrischen Körpers (5) in Strömungsverbindung steht,
**daß** der zylindrische Körper (5) ein Innengewinde (10) aufweist und
**daß** das Außengewinde (11) des Luer-Lock-Gegenstückes (2) und das Innengewinde (10) des zylindrischen Körpers (5) im verschraubtem Zustand flüssigkeitsdurchlässig sind.

2. Medizinischer Gegenstand und Vorrichtung nach Anspruch 1, **dadurch gekennzeichnet,**
**daß** im Innenraum (7) des zylindrischen Körpers (5) ein Luer-Kegel (3) angeordnet ist, der in unmittelbarer Strömungsverbindung mit dem Kanal (4) steht, und daß der Luer-Kegel eine radiale Öffnung (13) aufweist, die den Kanal (4) mit dem Innenraum (7) des zylindrischen Körpers (5) verbindet.

3. Medizinischer Gegenstand und Vorrichtung nach Anspruch 1, **dadurch gekennzeichnet,**
**daß** der Kanal (4) mit dem Innenraum (7) des zylindrischen Körpers (5) unmittelbar in Strömungsverbindung steht.

4. Medizinischer Gegenstand und Vorrichtung nach einem der Ansprüche 1 bis 3, **dadurch gekennzeichnet,**
**daß** der zylindrische Körper (5) mindestens eine radiale Öffnung (14, 16) zum Abfluß von Flüssigkeit aus dem Innenraum (7) aufweist.

5. Medizinischer Gegenstand und Vorrichtung nach Anspruch 4, **dadurch gekennzeichnet,**
**daß** eine radiale Öffnung (16) am oberen Rand (15) des zylindrischen Körpers angebracht ist.

## Claims

1. A medical article in the form of a luer lock counterpart (2) having an external thread (11), and a device for the cleaning thereof, the device having a cylindrical body (5), **characterised in that** the device (1) has a channel (4) opening into the cylindrical body (5), via which channel (4) cleaning fluid can be supplied, the channel (4) being in a flow connection with the interior of the cylindrical body (5), **in that** the cylindrical body (5) has an internal thread (10) and **in that** the external thread (11) of the luer lock counterpart (2) and the internal thread (10) of the cylindrical body (5) are permeable to fluid when screwed together.

2. A medical article and a device according to claim 1, **characterised in that** a luer cone (3) is arranged in the interior (7) of the cylindrical body (5) and is in a direct flow connection with the channel (4), and **in that** the luer cone has a radial opening (13) connecting the channel (4) to the interior (7) of the cylindrical body (5).

3. A medical article and a device according to claim 1, **characterised in that** the channel (4) is in a direct flow connection with the interior (7) of the cylindrical body (5).

4. A medical article and a device according to any one of claims 1 to 3, **characterised in that** the cylindrical body (5) has at least one radial opening (14, 16) for the discharge of fluid from the interior (7).

5. A medical article and a device according to claim 4, **characterised in that** a radial opening (16) is provided at the upper edge (15) of the cylindrical body.

## Revendications

1. Objet médical en forme de connecteur de type luer-lock (2), lequel présente un filetage extérieur (11), et dispositif pour le nettoyage de cet objet, le dispositif présentant un corps cylindrique (5), **caractérisé en ce que**
le dispositif (1) présente un canal (4) qui aboutit dans le corps cylindrique (5), par le biais duquel du liquide de nettoyage peut être amené, le canal (4) étant en liaison d'écoulement avec l'espace intérieur du corps cylindrique (5),
le corps cylindrique (5) présente un filetage intérieur (10) et
le filetage extérieur (11) du connecteur de type luer-lock (2) et le filetage intérieur (10) du corps cylindrique (5) sont perméables aux liquides lorsqu'ils sont vissés.

2. Objet médical et dispositif selon la revendication 1, **caractérisé en ce**
**qu'**un cône luer (3) qui est en liaison d'écoulement directe avec le canal (4) est disposé dans l'espace intérieur (7) du corps cylindrique (5), et en ce que le cône luer présente un orifice radial (13), qui relie le canal (4) à l'espace intérieur (7) du corps cylindrique (5).

3. Objet médical et dispositif selon la revendication 1, **caractérisé en ce**
**que** le canal (4) est en liaison d'écoulement directe avec l'espace intérieur (7) du corps cylindrique (5).

4. Objet médical et dispositif selon l'une quelconque des revendications 1 à 3, **caractérisé en ce**
**que** le corps cylindrique (5) présente au moins un orifice radial (14, 16) pour l'écoulement de liquide en dehors de l'espace intérieur (7).

5. Objet médical et dispositif selon la revendication 4, **caractérisé en ce**
**qu'**un orifice radial (16) est monté sur le bord supérieur (15) du corps cylindrique.
